**Europäisches Patentamt**

**European Patent Office**

**Office européen des brevets**

(19)

(11) Veröffentlichungsnummer: **0 093 297**
**B1**

(12)

# EUROPÄISCHE PATENTSCHRIFT

(45) Veröffentlichungstag der Patentschrift:
**18.12.85**

(51) Int. Cl.⁴: **G 03 B 42/02,** A 61 B 6/00

(21) Anmeldenummer: **83103684.3**

(22) Anmeldetag: **15.04.83**

(54) **Röntgenuntersuchungsgerät.**

(30) Priorität: **30.04.82 DE 3216216**

(43) Veröffentlichungstag der Anmeldung:
**09.11.83 Patentblatt 83/45**

(45) Bekanntmachung des Hinweises auf die Patenterteilung:
**18.12.85 Patentblatt 85/51**

(84) Benannte Vertragsstaaten:
**DE FR**

(56) Entgegenhaltungen:
**US - A - 4 032 789**
**US - A - 4 097 748**
**US - A - 4 139 776**

(73) Patentinhaber: **Siemens Aktiengesellschaft, Berlin und München Wittelsbacherplatz 2, D-8000 München 2 (DE)**

(72) Erfinder: **Seuss, Eckard,
Johann-Sebastian-Bach-Strasse 5, D-8523 Baiersdorf (DE)**
Erfinder: **Seubert, Hans-Peter, Untere Hauptstrasse 37, D-8551 Heroldsbach (DE)**

ACTORUM AG

Beschreibung

Die Erfindung betrifft ein Röntgenuntersuchungsgerät gemäss dem Oberbegriff des Patentanspruches 1.

Es ist ein Röntgenuntersuchungsgerät bekannt (DE-U Nr. 7710947), bei dem das Röntgenstrahlenbündel schlitzförmig eingeblendet über das Untersuchungsobjekt hinweg bewegt wird. Bei diesem Röntgenuntersuchungsgerät ist hinter dem Untersuchungsobjekt und unmittelbar vor der Filmebene eine weitere Schlitzblende vorgesehen, die synchron mit dem schlitzförmig eingeblendeten Strahlenbündel und zu diesem ausgerichtet über die Filmebene hinweg bewegt wird. Bei einem solchen Röntgenuntersuchungsgerät ist der Streustrahlenanteil, der auf die Röntgenaufnahme fällt, besonders gering. Die Detailerkennbarkeit ist entsprechend gut. Für eine korrekte Belichtung des Filmblattes und eine Minimalisierung des Streustrahlenanteiles ist jedoch eine exakte Synchronisation zwischen dem schlitzförmig eingeblendeten Strahlenbündel und der filmnahen Schlitzblende erforderlich. Bei dem vorbekannten Röntgenuntersuchungsgerät ist der Stelltrieb für die Bewegung des Schlitzes in der Primärstrahlenblende über einen Positionsvergleicher mit dem Stelltrieb für die filmnahe Schlitzblende verbunden, so dass beide Schlitze synchron zueinander ausgerichtet bewegt werden. Bei dieser Konstruktion ist der Aufwand für die synchrone Bewegung der beiden Schlitzblenden in erheblichem Masse von der erforderlichen Genauigkeit abhängig. Er ist bei der im vorliegenden Fall zu fordernden Genauigkeit der Nachführung beträchtlich.

Der Erfindung liegt die Aufgabe zugrunde, einen Weg zu weisen, wie die hinreichend genaue synchrone Bewegung der beiden Schlitzblenden mit geringstmöglichem Aufwand durchgeführt werden kann.

Bei einem Röntgenuntersuchungsgerät gemäss dem Oberbegriff des Patentanspruches 1 ist diese Aufgabe erfindungsgemäss durch die im kennzeichnenden Teil dieses Anspruches angegebenen Ausbildung gelöst. Bei dieser Lösung kann mit handelsüblichen digitalen Bauelementen gearbeitet werden. Auch lässt sich so die Steuergenauigkeit durch höhere Impulszahlen pro Wegeinheit in weiten Grenzen an die jeweils geforderte Steuergenauigkeit anpassen.

Bei der Ausgestaltung der Erfindung gemäss dem Patentanspruch 2 ist der Stelltrieb relativ einfach zu steuern. Bei der Ausgestaltung gemäss dem Patentanspruch 3 erübrigt sich eine besondere, den Winkelfehler kompensierende Signalverarbeitung. Die Weiterbildung der Erfindung gemäss dem Patentanspruch 4 ergibt eine weitere Vereinfachung des Stelltriebes. Dafür ist eine Steuerung des Untersetzers erforderlich. Letztere ist jedoch mit handelsüblichen preiswerten digitalen Bauelementen aufgrund der insgesamt aufgelaufenen Impulse möglich.

Weitere Einzelheiten der Erfindung werden anhand eines in den Figuren dargestellten Ausführungsbeispieles erläutert. Es zeigen:

Fig. 1 eine schaubildliche Seitenansicht des Röntgenuntersuchungsgerätes,

Fig. 2 eine vergrösserte Darstellung eines den Winkelfehler der Primärstrahlenblende kompensierenden Getriebes, und

Fig. 3 eine vergrösserte Darstellung eines anderen Getriebes für die Schwenkung der Primärstrahlenblende.

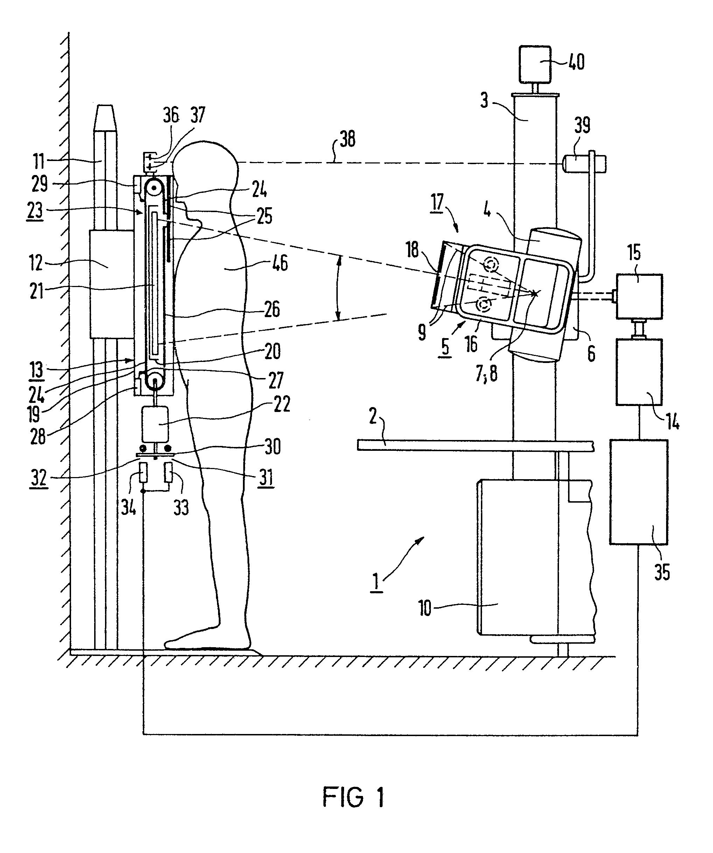

In der Fig. 1 ist ein Röntgenuntersuchungsgerät 1 mit einer horizontalen Patientenlagerungsplatte 2 und einer an einer neben der Patientenlagerungsplatte angeordneten Stativsäule 3 höhenverstellbaren Röntgenröhre 4 angedeutet. Die Röntgenröhre, an der eine Primärstrahlenblende 5 angeflanscht ist, ist an einem längs der Stativsäule 3 verschiebbaren Hubwagen 6 befestigt. Sie ist um eine horizontale, durch den Brennpunkt 7 gehende Achse 8 drehbar und mit ihrem Strahlenkegel 9 wahlweise auf die Patientenlagerungsplatte 2 und einem unter der Patientenlagerungsplatte angedeuteten Bildverstärker 10 oder einem unmittelbar unter der Patientenlagerungsplatte einschiebbaren Kassettenhalter (nicht dargestellt), oder auf ein neben dem Röntgenuntersuchungsgerät 1 an einem Wandstativ 11 an einem höhenverstellbaren Stativwagen 12 befestigten Aufnahmeeinrichtung 13 ausrichtbar. Abgesehen von dieser Drehung um in hier nicht weiter dargestellter Weise vorgegebene 90° ist die Röntgenröhre 4 mitsamt der angeflanschten Primärstrahlenblende 5 mittels eines aus einem Schrittmotor 14 und angeflanschten Getriebe 15 bestehenden Stelltrieb drehbar. Am Gehäuse 16 der Primärstrahlenblende 5 ist eine Schlitzblende 17 so aufsteckbar, dass ihr Schlitz 18 parallel zur Schwenkachse 8 ausgerichtet ist.

In der Darstellung der Fig. 1 ist das Gehäuse 19 der Aufnahmeeinrichtung 13 aufgeschnitten dargestellt. Man erkennt in ihr einen Bildschichtträger 20 mit einer Röntgenfilmkassette 21 sowie einen von zwei von einem Antriebsmotor 22 angetriebenen endlosen Seilzügen 23, an deren einen Trumm 24 eine geschlitzte Blendenplatte 25 parallel zur Bildschichtebene, d.h. der Ebene des in der Röntgenfilmkassette 21 befindlichen Filmblattes (nicht dargestellt) verschiebbar ist. Am anderen Trumm 26 des einen der beiden Seilzüge ist eine Nocke 27 befestigt, die in den beiden Extremstellungen der geschlitzten Blendenplatte 25 mit jeweils einem Nockenschalter 28, 29 in Eingriff bringbar ist. Die Achse des Antriebsmotors 22 trägt ausserdem eine Lochscheibe 30, die zwischen zwei Lichtschranken 31, 32 hindurchdrehbar ist. Die Ausgänge der beiden Fotodetektoren 33, 34 der beiden Lichtschranken 31, 32 sind an einen Untersetzer 35 angeschlossen. Dessen Ausgang ist seinerzeit wiederum mit dem Schrittmotor 14 des Stelltriebs für die Primärstrahlenblende 5 verbunden. Ausserdem erkennt man am Gehäuse 19 der Aufnahmeeinrichtung zwei nebeneinander angeordnete Sensoren 36, 37, auf die der Pilotstrahl 38 eines separaten am Hubwagen 6 der Röntgenröhre 4 befestigten Punktstrahlers 39 gerichtet ist. Beide Sensoren 36, 37 sind in hier nicht weiter dargestellter Weise über eine Nachlaufsteuerung an eine den Hubwagen 6 der Röntgenröhre 4 in der Höhe verstellenden Hilfsantrieb 40 angeschlossen.

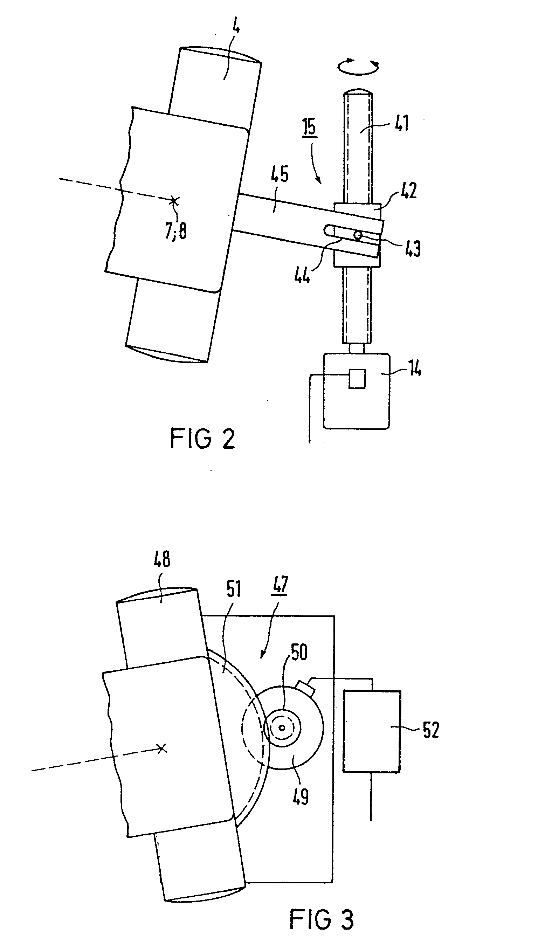

Die Fig. 2 zeigt den Aufbau des Antriebes 14, 15 für die Drehung der Röntgenröhre 4. Er besteht aus dem Schrittmotor 14, welcher eine Spindel 41 dreht, sowie aus einer an der Spindel 41 beim Drehen derselben höhenverstellbaren Mutter 42, die einen Zapfen 43 trägt, der in einen Längsschlitz 44 eines Hebels 45 eintaucht, welcher verdrehungssicher mit der Röntgenröhre 4 verbunden ist.

Soll ein Patient 46 nach dem Schlitzaufnahmeverfahren untersucht werden, so wird die Röntgenröhre 4 so gedreht, dass ihr Strahlenkegel 9 horizontal auf die Aufnahmeeinrichtung 13 ausgerichtet ist und wird die Schlitzblende 17 auf die Primärstrahlenblende 5 aufgesteckt. Wird jetzt der Punktstrahler 39 eingeschaltet und die Aufnahmeeinrichtung 13 mit dem Stativwagen 12 höhenverstellt, so wird der Hilfsantrieb 40 eingeschaltet, sobald wenigstens einer der beiden Sensoren 36, 37 vom Pilotstrahl 38 des Punktstrahlers 39 getroffen worden ist. Der Hilfsantrieb 40 an der Stativsäule 3 des Röntgenuntersuchungsgerätes 1 wird, je nach dem, welcher der beiden Sensoren 36, 37 zuletzt vom Pilotstrahl 38 getroffen worden ist, solange in einem Drehsinn eingeschaltet, der den Hubwagen in der Höhe nachführt, bis der Pilotstrahl beide Sensoren gleichstark trifft. Sobald dies der Fall ist, ist die Primärstrahlenblende 5 bei horizontalem Strahlengang exakt auf die Aufnahmeeinrichtung 13 ausgerichtet.

In der Aufnahmeeinrichtung 13 befindet sich die geschlitzte Blendenplatte 25 in einer durch den unteren Nockenschalter 28 vorgegebenen Extremlage. Zur Ausrichtung der Primärstrahlenblende samt der aufgesteckten Schlitzblende 17 auf den Schlitz der geschlitzten Blendenplatte 25 der Aufnahmeeinrichtung 13 ist dem Untersetzer 35 eine vorgegebene Impulsfolge eingeprägt, die vor dem Auslösen der eigentlichen Röntgenaufnahme den Schrittmotor 14 soweit dreht, dass er die Röntgenröhre 4 mitsamt der Primärstrahlenblende 5 um einen Winkel schwenkt, der den Abstand der Extremlage der geschlitzten Blendenplatte 25 zum Fusspunkt der Mittelsenkrechten auf die Aufnahmeeinrichtung 13 entspricht.

Beim Auslösen der Röntgenaufnahme verfährt der Antriebsmotor 22 der Aufnahmeeinrichtung 13 die geschlitzte Blendenplatte 25 parallel zur Bildschichtebene. Er wird abgeschaltet, sobald die geschlitzte Blendenplatte ihre untere Extremlage erreicht hat bzw. die am Seilzug 23 befestigte Nocke 27 den oberen Nockenschalter 29 betätigt hat. Während der Verstellung der geschlitzten Blendenplatte 25 dreht der Antriebsmotor 22 die auf seiner Achse befestigte Lochscheibe 30 zwischen Lichtquelle und Fotodetektor der beiden Lichtschranken 31, 32 durch. Die von den Fotodetektoren 33, 34 der beiden Lichtschranken 31, 32 erzeugten Impulse werden im Untersetzer 35 untersetzt und an den Schrittmotor 14 weitergeleitet. Letzterer dreht die Spindel 41 und führt über den mit der Spindelmutter 42 in Eingriff befindlichen Hebel 45 die Röntgenröhre mitsamt der Primärstrahlenblende nach, so dass der schlitzförmig eingeblendete Strahlenkegel stets auf den Schlitz der geschlitzten Blendenplatte 25 ausgerichtet bleibt. Über die beiden Nockenschalter 28, 29 wird der Untersetzer 35 nach jeder Aufnahme erneut auf Null gesetzt. Hierdurch werden Zählfehler vermieden. Das Untersetzungsverhältnis des Untersetzers kann bei diesem Getriebe 15 konstant sein.

Die Fig. 3 zeigt einen anderen Antrieb 47 für die Drehung der Röntgenröhre 48. Hier treibt der Schrittmotor 49 ein Ritzel 50 an, welches mit einem an der Röntgenröhre 48 verdrehungssicher befestigten Zahnrad 51 kämmt. Da bei diesem Getriebe jedem Schritt des Schrittmotors 49 ein gleichgrosser Winkelschritt der Röntgenröhre 48 entspricht, muss anders als beim Ausführungsbeispiel der Fig. 1 und 2 der Untersetzer 52 zur Kompensation des sonst entstehenden Winkelfehlers vorprogrammiert sein und sein Untersetzungsverhältnis in Abhängigkeit von der Lage der geschlitzten Blendenplatte 25 in der Aufnahmeeinrichtung 13 bzw. von der Zahl der insgesamt vom Start aus einer Extremlage bisher aufgelaufenen Impulszahlen ändern. Es wäre auch möglich, statt des vorprogrammierten Untersetzers 52 einen Mikroprozessor (nicht dargestellt) zu verwenden, der das Untersetzungsverhältnis in der Abhängigkeit von der Stellung der geschlitzten Blendenplatte 25 in der Aufnahmeeinrichtung bzw. der Gesamtzahl der bisher eingegangenen Impulse verändert.

## Patentansprüche

1. Röntgenuntersuchungsgerät mit einer an einer Röntgenröhre befestigten Primärstrahlenblende mit schlitzförmiger Einblendung, mit einem Bildschichtträger, mit einer in Strahlenrichtung unmittelbar vor der Bildschicht verschiebbaren schlitzförmigen Einblendvorrichtung, mit einem Antrieb für die Verschiebung des Schlitzes der Einblendvorrichtung parallel zur Bildschicht und senkrecht zu seiner Ausrichtung, gekennzeichnet durch Stellmittel zur fortlaufenden synchronen Ausrichtung der Einblendung der Primärstrahlenblende auf den Schlitz der Einblendvorrichtung, wobei ein wegabhängig gesteuerter Impulsgeber (31 bis 34) an den Antrieb (22) für die Einblendvorrichtung (25) angeschlossen ist und der Stelltrieb (14, 15; 41 bis 45; 47, 49 bis 51) für die Primärstrahlenblende (5) über den Impulsgeber und einen Pulsuntersetzer (35; 52) gesteuert wird.

2. Röntgenuntersuchungsgerät nach Anspruch 1, dadurch gekennzeichnet, dass die Primärstrahlenblende (5) von einem Schrittmotor (14, 49) mit einem nachgeschalteten Getriebe (15, 41 bis 45, 47, 50, 51) geschwenkt wird.

3. Röntgenuntersuchungsgerät nach Anspruch 2, dadurch gekennzeichnet, dass der Stellweg des Schrittmotors (14) linear proportional zur Zahl der vom Pulsuntersetzer (35) abgegebenen Impulse ist und das Getriebe (15) den Winkelfehler kompensiert.

4. Röntgenuntersuchungsgerät nach Anspruch 1, dadurch gekennzeichnet, dass der Stelltrieb (47, 49 bis 51) den Schwenkhebel der Primärstrahlenblende linear proportional zur Zahl der vom Pulsuntersetzer (52) abgegebenen Impulse verändert und das Untersetzungsverhältnis des Pulsuntersetzers (52) von der Position der Einblendvorrichtung (25) abhängig ist.

5. Röntgenuntersuchungsgerät nach Anspruch 4, dadurch gekennzeichnet, dass ein durch die Zahl der Impulse programmgesteuerter Pulsuntersetzer (52) zur Kompensation des Winkelfehlers in den Impulsweg eingesetzt ist.

6. Röntgenuntersuchungsgerät nach Anspruch 4, dadurch gekennzeichnet, dass zur Kompensation des Winkelfehlers in den Impulsweg ein das Untersetzungsverhältnis des Pulsuntersetzers in Abhängigkeit vom Stellweg der Einblendvorrichtung (25) ändernder Mikroprozessor eingesetzt ist.

7. Röntgenuntersuchungsgerät nach Anspruch 1, dadurch gekennzeichnet, dass der Primärstrahlenblende (5) eine einen Pilotstrahl (38) erzeugende Strahlenquelle (39) zugeordnet ist, dass die den Bildschichtträger enthaltende Aufnahmeeinrichtung (13) mindestens zwei Sensoren (36, 37) für den Pilotstrahl trägt, und dass an die Sensoren ein Hilfsantrieb (40) für die Höhennachführung der Schwenkachse (8) der Einheit Röntgenröhre – Primärstrahlenblende an die Höhenposition der Aufnahmeeinrichtung (13) angeschlossen ist.

## Claims

1. An X-ray examination apparatus having a primary radiation diaphragm secured to an X-ray tube and having a picture layer carrier with a slotted adjusting device which can be directly displaced in front of the picture layer in the beam direction, having a drive for the displacement of the slots of the adjusting device parallel to the picture layer and at right angles to the alignment, characterised by setting means for the continuous synchronous alignment of the adjustment of the primary radiation diaphragm onto the slot of the adjusting device, where a pulse generator (31 to 34) controlled in dependence upon the path is connected to the drive (22) for the adjusting device (25), and the setting drive (14, 15; 41 to 45; 47, 49 to 51) for the primary radiation diaphragm (5) is controlled by means of the pulse generator and a pulse step-down device (35; 52).

2. An X-ray examination apparatus as claimed in Claim 1, characterised in that the primary radiation diaphragm (5) is swivelled by a stepping motor (14, 49) with a following gear unit (15, 41 to 45, 47, 50, 51).

3. An X-ray examination apparatus as claimed in Claim 2, characterised in that the setting path of the stepping motor (14) is linearly proportional to the number of the pulses emitted by the pulse step-down device (35) and the gear unit (15) compensates the angle error.

4. An X-ray examination apparatus as claimed in Claim 1, characterised in that the setting drive (47, 49 to 51) changes the swivel lever of the primary radiation diaphragm so as to be linearly proportional to the number of pulses emitted by the pulse step-down device (52), and the reducing gear ratio of the pulse step-down device (52) is dependent upon the position of the adjusting device (25).

5. An X-ray examination apparatus as claimed in Claim 4, characterised in that a pulse step-down device (52) programme-controlled by the number of pulses is inserted into the pulse path for the compensation of the angle error.

6. An X-ray examination apparatus as claimed in Claim 4, characterised in that a microprocessor which changes the reducing gear ratio of the pulse step-down device in dependence upon the setting path of the adjusting device (25) is inserted into the pulse path in order to compensate the angle error.

7. An X-ray examination apparatus as claimed in Claim 1, characterised in that the primary radiation diaphragm (5) is assigned a radiation source (39) which produces a pilot beam (38), that the accommodating device (13) which comprises the picture layer carrier supports at least two sensors (36, 37) for the pilot beam, and that an auxiliary drive (40) for the height follow-up device of the swivel axis (8) of the X-ray unit primary radiation diaphragm is connected at the height position of the accommodating device (13).

## Revendications

1. Appareil pour l'examen radiologique, avec un diaphragme des rayons primaires à diaphragmation en forme de fente, fixé au tube à rayons X, avec un support de couche d'image, avec un dispositif de diaphragmation en forme de fente, déplaçable devant la couche d'image, directement dans la direction des rayons, avec une commande pour le déplacement de la fente du dispositif de diaphragmation, parallèlement à la couche d'image et perpendiculairement au sens de son orientation, caractérisé par des moyens de réglage pour orienter de façon continue et synchrone la diaphragmation du diaphragme des rayons primaires sur la fente du dispositif de diaphragmation, un générateur d'impulsions (31 à 34) commandé en fonction de la course étant relié à la commande (22) pour le dipositif de diaphragmation (25), et le mécanisme de réglage (14, 15; 41 à 45; 47, 49 à 51) pour le diaphragme (5) des rayons primaires étant commandé par l'intermédiaire du générateur d'impulsions ou d'un diviseur d'impulsions (35; 52).

2. Appareil pour l'examen radiologique selon la revendication 1, caractérisé par le fait que le diaphragme des rayons primaires (5) est basculé à l'aide d'un moteur pas-à-pas (14, 49), avec une transmission aval (15, 41 à 45, 47, 50, 51).

3. Appareil pour l'examen radiologique selon la revendication 2, caractérisé par le fait que la course de réglage du moteur pas-à-pas (14) est linéairement proportionnelle au nombre des impulsions fournies par le diviseur d'impulsions (35), alors que la transmission (15) compense le défaut angulaire.

4. Appareil pour l'examen radiologique selon la revendication 1, caractérisé par le fait que le dispositif de réglage (41, 49 à 51) modifie le levier de basculement du diaphragme des rayons primaires linéairement et proportionnellement au nombre des impulsions fournies par le diviseur d'impulsions (52) et que le rapport de démultiplication du diviseur d'impulsions (52) dépend de la position du dispositif de diaphragmation (25).

5. Appareil pour l'examen radiologique selon la revendication 4, caractérisé par le fait qu'un diviseur d'impulsions (52), commandé par programme en fonction du nombre des impulsions, est mis en œuvre dans la voie de transmission des impulsions pour compenser une erreur angulaire.

6. Appareil pour l'examen radiologique selon la revendication 4, caractérisé par le fait que pour compenser l'erreur angulaire on prévoit dans la voie de transmission des impulsions un microprocesseur qui modifie le rapport de démultiplication du diviseur d'impulsions en fonction de la course de réglage du dispositif de diaphragmation (25).

7. Appareil pour l'examen radiologique selon la revendication 1, caractérisé par le fait qu'au diaphragme des rayons primaires (5) est associée une source de radiations (39) qui produit un rayon-pilote (38), que le dispositif de prise de vue (13) qui contient le support de couche d'image y porte deux détecteurs (36, 37) pour le rayon-pilote, et qu'aux détecteurs est reliée une commande auxiliaire (40) pour l'asservissement en hauteur de l'axe de basculement (8) de l'unité tube à rayons X-diaphragme des rayons primaires, en fonction de la position en hauteur du dispositif de prise de vue (13).

FIG 1

FIG 2

FIG 3